# EUROPEAN PATENT APPLICATION

(11) **EP 3 001 192 A1**
(43) Date of publication of application: **30.03.2016**
(21) Application number: 14186030.4
(22) Date of filing: 23.09.2014
(51) Int. Cl.: G01N 33/00

(54) **An apparatus and associated methods for sensing composition of a fluid analyte flow**

(71) Applicant: Nokia Technologies OY, 02610 Espoo (FI)
(72) Inventor: Borini, Stefano Marco, Cambridge, CB3 0FA (GB); White,, Richard, Huntingdon, PE28 9DY (GB)
(74) Representative: Potter Clarkson LLP

(57) **Abstract**

An apparatus (100) comprising a fluid analyte flow modulator (102) and a sensing element (104),
the apparatus configured such that the fluid analyte flow modulator (102) provides for a temporal modulation (108) of a fluid analyte flow (110) directed to the sensing element (104);
the sensing element (104) configured to provide detectable rising and decay sensor signalling based on temporally modulated interaction of one or more chemical species of the fluid analyte flow (110) with the sensor element (104), the detectable rising and decay sensor signalling representative of the composition of the fluid analyte flow (110).

## Description

### Technical Field

The present disclosure relates to the field of sensor devices configured to sense the composition of a fluid analyte flow, associated methods, computer programs and apparatus. Certain disclosed aspects/examples relate to portable electronic devices, in particular, so-called hand-portable electronic devices which may be hand-held in use (although they may be placed in a cradle in use). Such hand-portable electronic devices include so-called Personal Digital Assistants (PDAs), mobile telephones, smartphones and other smart devices, smartwatches, medical devices, navigation devices and tablet PCs.

### Background

A sensor device may provide signalling representative of the composition of a sensed fluid analyte.

The listing or discussion of a prior-published document or any background in this specification should not necessarily be taken as an acknowledgement that the document or background is part of the state of the art or is common general knowledge. One or more aspects/examples of the present disclosure may or may not address one or more of the background issues.

### Summary

In a first aspect there is provided an apparatus comprising:
a fluid analyte flow modulator and a sensing element,
the apparatus configured such that the fluid analyte flow modulator provides for a temporal modulation of a fluid analyte flow directed to the sensing element;
the sensing element configured to provide detectable rising and decay sensor signalling based on temporally modulated interaction of one or more chemical species of the fluid analyte flow with the sensor element, the detectable rising and decay sensor signalling representative of the composition of the fluid analyte flow.

Analysis of the dynamic response (rising and decay signalling) of the sensing element to the modulated fluid analyte flow may provide more information about the composition of the fluid analyte than the steady state response of the same sensing element to a static sample of the same fluid analyte.

The sensing element may be configured to provide the detectable rising and decay sensor signalling based on adsorption of one or more chemical species of the fluid analyte flow by the sensing element.

Transient adsorption of a chemical species onto the surface of a sensor may transiently change the behaviour of the sensor and thereby provide for detectable signalling representative of the composition of the fluid analyte.

The sensing element may be configured to provide the detectable rising and decay sensor signalling based on van der Waals' interactions between the sensing element and one or more chemical species of the fluid analyte flow.

Interactions between particular chemical species and a sensing element based on van der Waals' force may provide a dynamically evolving changes to the sensor that evolve based on modulation of a flow of fluid analyte comprising the particular chemical species in a way that may be representative of the composition fluid analyte.

The sensing element may comprise a plurality of sensors configured to provide a plurality of respective detectable rising and decay sensor signals, each sensor configured to provide a particular detectable rising and decay sensor signalling based on temporally modulated interaction of one or more chemical species of the fluid analyte flow with the particular sensor, the plurality of detectable rising and decay sensor signals representative of the composition of the fluid analyte flow.

Multiple sensors may provide for more reliable detection of chemical species, for example by accumulating results from the respective sensors when a plurality of the same sensors is used.

Each sensor may be configured to provide the particular detectable rising and decay sensor signalling based on temporally modulated interaction of a particular different chemical species within the fluid analyte flow with the particular sensor. An array of sensors that each react differently to a particular chemical species may provide information about the presence of each chemical species in turn.

Each sensor may be configured to provide the particular detectable rising and decay sensor signalling with a particular different response time based on temporally modulated interaction of the one or more chemical species within the fluid analyte flow with the particular sensor. In some examples, the different sensors may react more or less quickly depending on the composition of a fluid analyte and based on the speed of reaction it may be possible to deduce the composition of the fluid analyte.

The fluid analyte flow modulator may be configured to provide for one or more discrete pulses of fluid analyte flow to the sensing element to provide for the temporally modulated interaction of the one or more chemical species of the fluid analyte flow with the sensor element. In some examples a single pulse of fluid analyte may be sufficient to provide sensor signalling to enable determination of the composition of the fluid analyte. In other examples a plurality of pulses of fluid analyte may provide sensor signally that contains more information than a single pulse, such as by improving signal to noise ratios by averaging.

The fluid analyte flow modulator may be configured to provide for a plurality of discrete pulses of fluid analyte flow to the sensing element to provide for a plurality of discrete temporally modulated interactions of the one or more chemical species of the fluid analyte flow with the sensor element, the plurality of discrete pulses such that rising and decay sensor signalling from the plurality of the temporally modulated interactions may be representative of the composition of the fluid flow.

The fluid analyte flow modulator may be configured to provide for a plurality of periodic pulses of fluid analyte flow to the sensing element to provide for a plurality of discrete temporally modulated interactions of the one or more chemical species of the fluid analyte flow with the sensor element, the plurality of periodic pulses such that a cumulative rising and decay sensor signalling from the plurality of discrete temporally modulated interactions may be representative of the composition of the fluid flow. Thus, in some examples, a periodic series of pulses may provide signalling that does not fully decay to the sensor original state before the introduction of a pulse of fluid analyte. The resulting cumulative signal may have a characteristic envelope, the shape of which may provide information about the composition of the fluid analyte.

The rising and decay sensor signalling representative of the composition of the fluid analyte flow may be representative of one or more of presence and concentration of the one or more chemical species. In some examples the response of a sensing element may be known to change when a particular chemical species is introduced to the sensor. Detection of such a change may reveal the presence of the chemical species concerned. In other examples the degree of change in the sensor response may be known to correlate with the concentration of the chemical species according to a particular relationship. Where this relationship is known the detection of a particular change in the sensor's response may enable identification of the presence of a particular concentration of the chemical species concerned.

The one or more chemical species may comprise one or more of water vapour and one or more of a Volatile Organic Compound. In some examples the response of a sensor to water vapour may change according to the concentration of a Volatile Organic Compound present. Detection of a particular change may thereby allow identification of the particular Volatile Organic Compound as being part of the composition of the fluid analyte.

The sensing element may comprise: a thin film of 2D material (e.g. graphene, graphene oxide, functionalised graphene, molybdenum sulphide or other transition metal dischalcogenides); a Field Effect Transistor; a biosensor; or one or more of: graphene, graphene oxide, functionalized graphene, and a metal dichalcogenide. In some examples sensors based on thin films of 2D material may be more sensitive to small changes in composition of a fluid analyte than sensors based on bulk materials.

The fluid analyte flow modulator may comprise a micro-electro-mechanical valve. In some examples a micro-electro-mechanical valve may enable precise control of the modulation of the flow of a fluid analyte.

According to a further aspect, there is provided a method comprising:
using/providing an apparatus, the apparatus comprising a fluid analyte flow modulator and a sensing element,
the apparatus configured such that the fluid analyte flow modulator provides for a temporal modulation of a fluid analyte flow directed to the sensing element;
the sensing element configured to provide detectable rising and decay sensor signalling based on temporally modulated interaction of one or more chemical species of the fluid analyte flow with the sensor element, the detectable sensor rising and decay signalling representative of the composition of the fluid analyte flow.

According to a further aspect, there is provided a computer program, the computer program comprising computer code to detect a signalling produced from an apparatus, the apparatus comprising a fluid analyte flow modulator and a sensing element,
the apparatus configured such that the fluid analyte flow modulator provides for a temporal modulation of a fluid analyte flow directed to the sensing element;
the sensing element configured to provide detectable rising and decay sensor signalling based on temporally modulated interaction of one or more chemical species of the fluid analyte flow with the sensor element, the detectable sensor rising and decay signalling representative of the composition of the fluid analyte flow.

Following the detection, the signalling can then be interpreted with respect to reference/control signalling to assess the composition of the fluid analyte flow. This is because different characteristics of rising and decay sensor signalling can be compared with corresponding characteristics in reference/control signalling established from control experiments.

According to a further aspect, there is provided a computer program, the computer program comprising computer code for controlling the fluid analyte flow modulator of an apparatus, the apparatus comprising the fluid analyte flow modulator and a sensing element, the apparatus configured such that the fluid analyte flow modulator provides for a temporal modulation of a fluid analyte flow directed to the sensing element;
the sensing element configured to provide detectable rising and decay sensor signalling based on temporally modulated interaction of one or more chemical species of the fluid analyte flow with the sensor element, the detectable sensor rising and decay signalling representative of the composition of the fluid analyte flow.

The steps of any method disclosed herein do not have to be performed in the exact order disclosed, unless explicitly stated or understood by the skilled person.

Corresponding computer programs (which may or may not be recorded on a carrier) for implementing one or more of the methods disclosed herein are also within the present disclosure and encompassed by one or more of the described example embodiments.

The present disclosure includes one or more corresponding aspects, examples or features in isolation or in various combinations whether or not specifically stated (including claimed) in that combination or in isolation. Corresponding means and corresponding functional units or performing one or more of the discussed functions are also within the present disclosure.

The above summary is intended to be merely exemplary and non-limiting.

### Brief Description of the Figures

A description is now given, by way of example only, with reference to the accompanying drawings, in which:
Figure 1a illustrates an example apparatus embodiment comprising a fluid analyte flow modulator and a sensing element;
Figure 1b illustrates a detectable rising and decay sensor signalling provided by the sensing element based on temporally modulated interaction of the sensing element with a modulated fluid analyte flow;
Figure 2 illustrates interaction of a fluid analyte with a sensing element based on a chemical species within the fluid analyte being adsorbed on to the surface of the sensing element;
Figure 3a illustrates a sensor based on a layered material in which the presence of a particular chemical species results in a particular layer spacing;
Figure 3b illustrates the sensor of figure 3a in which the presence of a different chemical species results in a different layer spacing;
Figure 3c illustrates a chart of experimental data showing the change in layer spacing of graphene oxide in the presence of water as a concentration of methanol is varied;
Figure 4a illustrates an example sensing element comprising a first sensor and a second senor;
Figure 4b illustrates a detectable rising and decay senor signalling provided by two different sensors in response to a modulated flow of a particular fluid analyte;
Figure 4c illustrates a different detectable rising and decay senor signalling provided by the sensors, disclosed in relation to figure 4b, in response to a modulated flow of a different fluid analyte;
Figure 5 illustrates a detectable rising and decay senor signalling provided by two different sensors with different response times to the same analyte;
Figure 6 illustrates an example fluid analyte flow modulator;
Figure 7a illustrates an example temporal modulation of a fluid analyte flow and a corresponding detectable rising and decay sensor signalling provide by a sensing element in response to the temporally modulated fluid analyte flow;
Figure 7b illustrates an example periodic temporal modulation of a fluid analyte flow and a corresponding cumulative detectable rising and decay sensor signalling provided by a sensing element in response to the periodic temporally modulated fluid analyte flow;
Figure 8a illustrates two different detectable rising and decay senor signals with different amplitudes provided by the same sensor in response to two separate modulated fluid analyte flows with different compositions;
Figure 8b illustrates two different detectable rising and decay senor signals with similar amplitudes provided by the same sensor in response to two separate modulated fluid analyte flows with different compositions;
Figure 8c illustrates a particular detectable rising and decay sensor signalling and certain characteristics of the signalling that may be representative of the composition of a modulated fluid analyte flow;
Figure 9 illustrates a chart of experimental data showing the Fast Fourier Transform magnitude of the response of a sensor as a function of frequency of a modulated fluid analyte flow comprising two different concentrations of acetone in the presence of water vapour.
Figure 10 illustrates an electronic device comprising an example apparatus embodiment of the present disclosure;
Figure 11 illustrates a flowchart according to a method of the present disclosure; and
Figure 12 illustrates schematically a computer readable medium providing a program.

### Description of Example Aspects

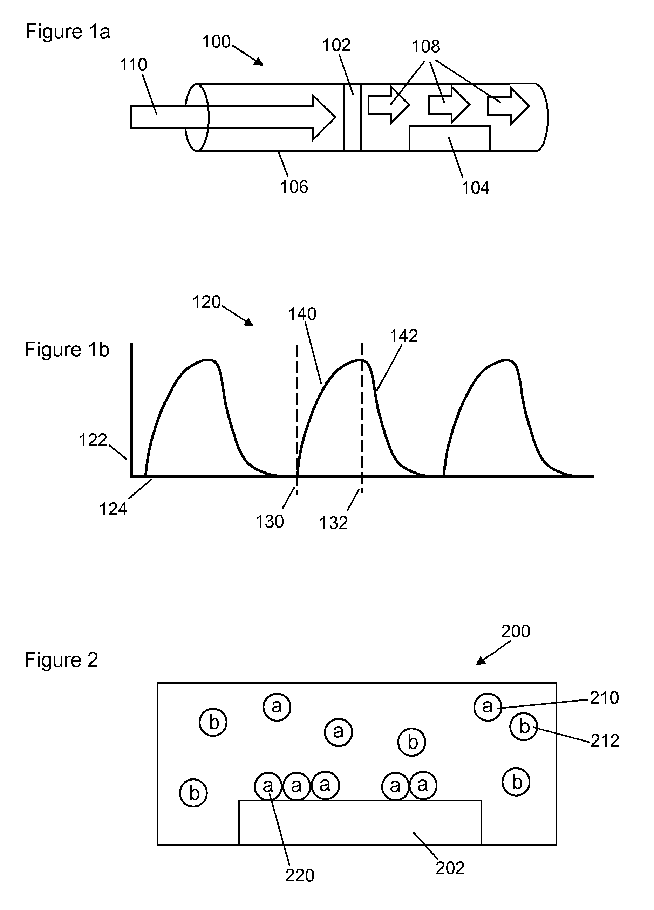

Certain embodiments disclosed herein may be considered to provide an apparatus 100, as illustrated in figure 1a, comprising a fluid analyte flow modulator 102 and a sensing element 104. In some examples the fluid analyte flow modulator 102 may comprise an envelope housing 106 configured to surround the sensing element 104 in order to direct a fluid analyte flow onto or past the sensing element 104. In other examples the sensing element 104 may be exposed to the ambient environment such that flow of fluid analyte onto the sensing element 104 rapidly dissipates into the environment.

The apparatus may be configured such that the fluid analyte flow modulator 102 provides for a temporal modulation 108 of a fluid analyte flow 110 directed to the sensing element 104. In some examples the flow modulator 102 may provide for a continuous smooth flow of fluid analyte with a time varying flow rate that varies between a lower flow rate and a higher flow rate. In other examples the flow modulator may provide a discrete/sharp pulse of fluid analyte flow in which the flow rate is approximately constant for a period of time and approximately zero for period of time before and after the pulse. Of course, in some examples the discrete pulses may not vary to an approximately zero value, but may go to a lower non-zero value.

The sensing element 104 may be configured to provide detectable rising and decay sensor signalling 120, as illustrated in figure 1b, based on temporally modulated interaction of one or more chemical species of the fluid analyte flow with the sensor element 104. Figure 1 b shows a chart 120 comprising the sensor signalling amplitude on a vertical axis and time on a horizontal axis 124. In some examples a pulse of fluid analyte flow directed to the sensing element 104 may result in an increasing interaction between the sensing element 104 and the fluid analyte as the pulse arrives at the sensing element 104 at an arrival time 130, followed by a decreasing or decaying interaction as the fluid analyte pulse departs or dissipates starting at a dissipation time 132. For a particular pulse, the increasing interaction may result in a rising sensor signalling 140. For the same pulse, the decreasing interaction may result in a decay sensor signalling 142.

The detectable rising and decay sensor signalling 120 may be representative of the composition of the fluid analyte flow. In some examples the speed of the response of the sensor may be indicative of the composition of a fluid analyte flow. The speed of response may relate to the time taken for a sensor to reach a saturation value of sensor signalling and/or the level of the saturation value. The speed of response may relate to the rate at which the sensor response decays to zero, or any other value relevant for a particular sensor in the absence of the fluid analyte. Since the dynamics of how a sensor responds may depend on the precise nature of the fluid analyte to which it is exposed, it may be possible to determine information about the composition of the fluid analyte by analysing the dynamic response of the sensor to that exposure.

Figure 2 illustrates an example apparatus 200 comprising a sensing element 202 where the sensing element 202 is configured to provide the detectable rising and decay sensor signalling based on adsorption of one or more chemical species of the fluid analyte flow by the sensing element. In this example two different chemical species, type 'a' 210 and type 'b' 212 are shown. In this example the sensing element 202 is configured to selectively adsorb the type 'a' chemical species, as shown where type 'a' species have been adsorbed 220 onto the surface of the sensing element 202.

In some examples the sensing element may be configured to provide the detectable rising and decay sensor signalling based on van der Waals' interactions between the sensing element and one or more chemical species of the fluid analyte flow. Van der Waals' interactions may provide for a relatively short lived bond being formed between the adsorbate and the adsorbent surface. The number of adsorbate particles may increase rapidly as a fluid analyte flow arrives at a sensor and then decline rapidly as the fluid analyte dissipates away from the sensor. The rapid change in the number of adsorbed particles and the effect on the sensor of those adsorbed particles may provide for a change in the sensor's output as a function of time. This dynamically changing output may provide a rising and decay sensor signalling suitable for determining the composition of the fluid analyte.

Certain types of sensor may be highly sensitive to adsorption of chemical species onto a sensor surface. In some examples, a sensor element may comprise a thin film of 2D material. 2D materials include graphene, graphene oxide, functionalized graphene, and metal dichalcogenide sheets. The large surface area of such 2D materials may provide for a large change in sensor behaviour when exposed to a fluid analyte containing particular chemical species. Furthermore, thin films of 2D materials can exhibit porosity that may enhance the interaction of the material with a fluid analyte or particular chemical species contained within the fluid analyte.

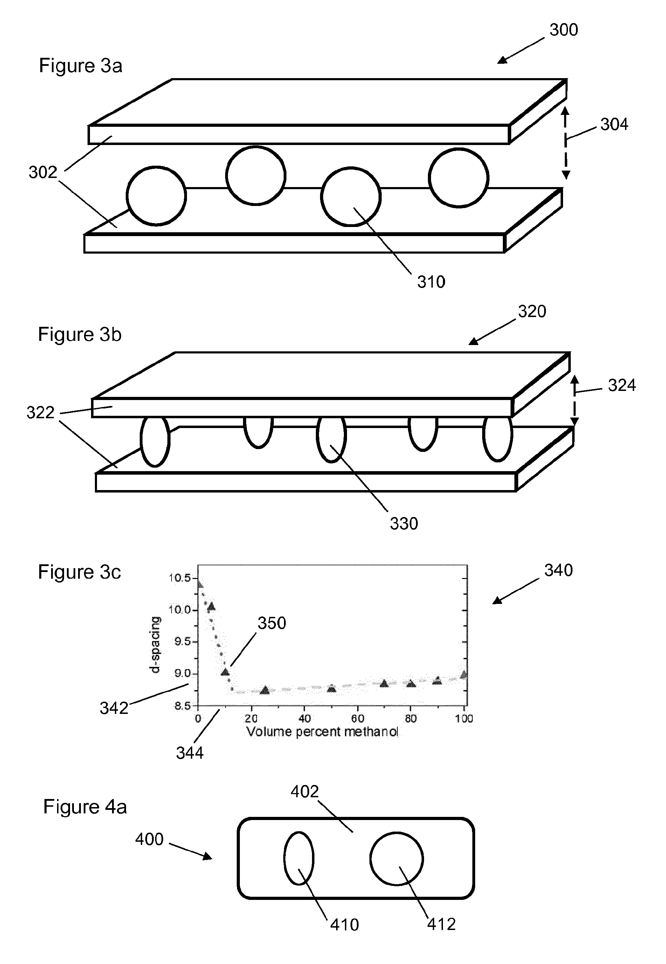

Figure 3a shows part of a sensor comprising layers of graphene oxide 302. Graphene oxide comprises a stack of layers made up of carbon and oxygen and hydrogen atoms. The layers 302 are spaced apart from each other with a particular inter-layer spacing 304. It is possible for some molecules to penetrate the spaces between adjacent layers. For example, water molecules 310 may diffuse between the layers 302.

Figure 3b shows part of as sensor 320 comprising layers of graphene oxide 322 that contain molecules of a Volatile Organic Compound 330. Examples of such compounds, that can penetrate graphene oxide, include methanol. The presence of methanol may significantly reduce the interlayer spacing 324.

Figure 3c illustrates experimental data 340 that shows measurements of interlayer spacing, on a vertical axis 342, for different concentrations of methanol dissolved in water, on a horizontal axis 344. Individual data points 350 are plotted on the chart and clearly show a strong dependence of the layer spacing on the concentration of methanol present. In general, the presence of particular chemical species between layers of graphene oxide or functionalized graphene oxide may change the interlayer spacing. Changes in interlayer spacing 324 may result in consequential changes in the physical behaviour of a graphene oxide sensor. For example, the impedance of the sensor, measured as a function of frequency, may change. The process of adsorbing methanol into a graphene oxide sensor may occur gradually over a finite period of time. Therefore, in response to a modulated fluid analyte flow comprising methanol, measurements of the electrical impedance of the sensor may provide rising and decay signalling. Direct Current (DC) and Alternative Current (AC) impedance measurement techniques can be used to give rise to the rising and decay signalling. Such dc impedance measurements (including resistance) may be conducted at a particular frequency, for example in the KHz range or in the MHz range, that is, at a frequency significantly higher than the frequency of the modulated fluid analyte flow (such that the sampling frequency electronics is much greater than the flow modulation frequency). In certain situations, it may be possible that AC measurements techniques would be used as DC techniques can polarise the material and cause drift for some sensing elements. A chart of the electrical impedance, measured at a particular frequency, as a function of time over the course of a modulation of the fluid analyte flow may therefore provide rising and decay signalling. Such changes in electrical impedance, or other detectable characteristics of the sensor, may enable the detection of methanol in the environment of the sensor.

The interaction between the graphene oxide sensor and water molecules, or other chemical species, may also change in the presence of methanol. Measurement of different responses of the graphene oxide to a given concentration of a particular chemical species in a particular fluid analyte may thereby enable to detection of the presence of methanol, or other Volatile Organic Compounds. In some examples the presence of a particular concentration of methanol may enhance or retard the adsorption of a particular chemical species into the sensor. Where the adsorption is retarded by the presence of methanol, the changes in sensor signalling may occur more slowly. A slower rising and decay sensor signalling, or other changes to the particular profile of the sensor response, based on changes to the non-linearity of the interaction between the sensor and a particular chemical species in the fluid analyte flow, may therefore provide information about the composition of the fluid analyte flow.

In some examples a sensor may comprise metal dichalcogenide sheets. Suitable examples of metal dichalcogenides may include molybdenum disulphide and tungsten diselenide.

In some examples a sensor element may comprise a Field Effect Transistor (FET). Adsorption of certain chemical species onto a surface of a FET may modify the switching time or the latency of the FET. Thereby, measurements of the switching time or latency of the FET may provide information about the composition of the fluid analyte.

In some examples a sensor element may comprise a biosensor. Such sensors are sensitive to the presence of biological components. Examples of sensitive biosensors include surface plasmon resonance sensors which may be configured to measure the dynamic change in a surface plasmon resonance in response to adsorption of material, such as biological components or chemical species, onto the surface of the sensor. Such sensing may be conducted by measuring changes in optical power of laser light absorbed by the resonance as the resonant conditions change in response to the adsorbate.

Figure 4a illustrates a sensing element 400 comprising a plurality of sensors configured to provide a plurality of respective detectable rising and decay sensor signals, each sensor may be configured to provide a particular detectable rising and decay sensor signalling based on temporally modulated interaction of one or more chemical species of the fluid analyte flow with the particular sensor, the plurality of detectable rising and decay sensor signals may be representative of the composition of the fluid analyte flow. In the example illustrated by figure 4a there are two sensors provided on the surface 402 of the sensing element 400: a first sensor 410 and spaced apart from the first sensor 410 a different second sensor 412. It will be appreciated that the plurality of sensors may comprise two or more sensors. In some examples the sensing element 400 may comprise tens of sensors. In other examples the sensing element 400 may comprise hundreds of sensors.

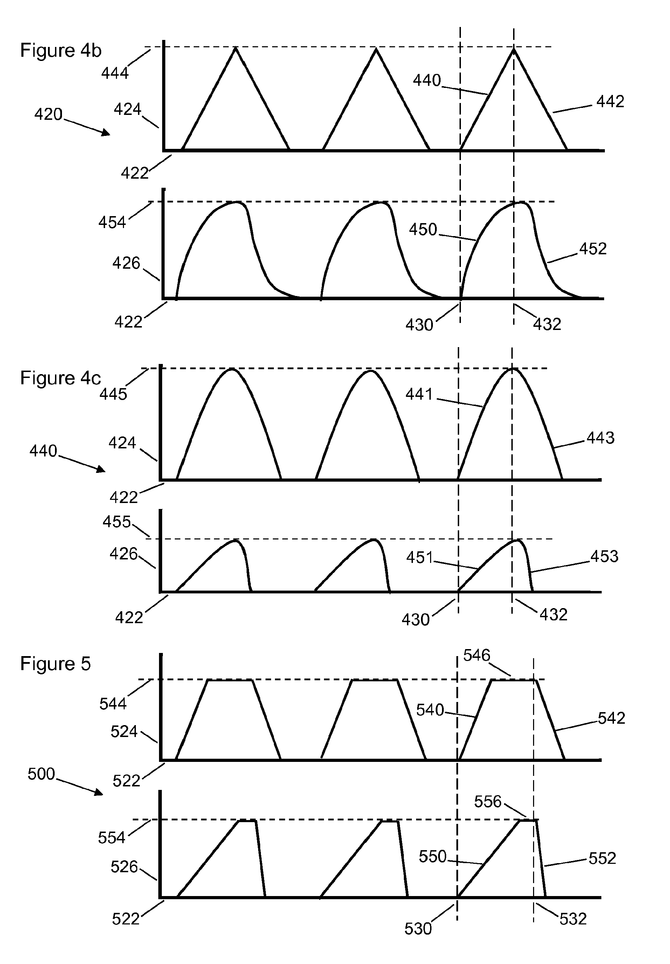

Figure 4b illustrates the detectable rising and decay sensor signalling 420 provided by two different sensors exposed to a temporally modulated flow of a particular fluid analyte. Signalling from each sensor is shown on separate charts in order to improve the clarity of the disclosure. The horizontal axis 422 of each chart displays the time that a signal is measured. It will be appreciated that measurements of each sensor are conducted simultaneously. A first vertical axis 424 relates to the amplitude of a first sensor signalling and a second vertical axis 426 relates to the amplitude of a second sensor signalling. In this example the sensor responses to three pulses of fluid analyte are shown, each response being essentially the same as earlier responses and each response decaying to zero before the next pulse of fluid analyte begins.

A particular pulse of a first fluid analyte begins at a first time 430 and ends at a second time 432. In response to the pulse, the first sensor provides a rising sensor signal 440. Once the pulse is complete, at the second time 432, the first sensor provides a decay sensor signal 442. The first sensor rising and decay sensor signal has a maximum amplitude 444 at the second time 432. It will be appreciated that in other examples a maximum amplitude may be achieved either before or after the end of a pulse of fluid analyte. In response to the pulse, the second sensor provides a rising sensor signal 450 and subsequently a decay sensor signal 452, with a maximum amplitude 454 at a certain time.

The response of the two sensors is quite different in form. While the first sensor signalling is approximately linear in both the rising 440 and decay 442 portions, the second sensor signalling is highly non-linear and quite different in shape as between the rising 450 portion and the decay 452 portion. In this example the different sensors provide a different response to the same fluid analyte flow and thereby provide different information about the composition of the first fluid analyte. In some examples, the relationship between a particular sensor response and the composition of a fluid analyte flow may be known based on characterisation of the particular sensor. In some examples, such as where a sensor comprises graphene oxide or functionalized graphene oxide it may be necessary to individually characterise each sensor because the exact molecular structure may vary from sensor to sensor.

Figure 4c illustrates the response of the same two sensors discussed above in relation to figure 4b when the two sensors are exposed to a series of pulses of a second fluid analyte flow of a different composition compared to the first fluid analyte. Features of figure 4c that are similar to figure 4b have been given similar reference numerals and will not necessarily be discussed in order to improve the clarity of the disclosure.

Figure 4c shows that the first sensor responds differently to the second fluid analyte flow, with a non-linear rising sensor signal 441 and a non-linear decay senor signal 443. In this example, the maximum amplitude 445 achieved by the sensor is similar to the maximum amplitude 444 achieve in response to the first fluid analyte flow. The second sensor also behaves differently, both with respect to the difference in the fluid analyte composition and with respect to the first sensor. In particular, the second sensor provides a different rising sensor signal 451, a different decay sensor signal 453 and achieves a significantly smaller maximum amplitude 455 than in response to the first fluid analyte. These differences may be used to determine the differences in the fluid analyte composition as between the first fluid analyte and the second fluid analyte. Determination of the composition of a fluid analyte flow may be based on pattern recognition of the rising and decay sensor signalling where the particular rising and decay sensor signalling corresponding to a particular composition of fluid analyte flow is known, either for a particular type of sensor or based on characterization of a particular individual sensor. The patterns of rising and decay sensor signalling indicative of different compositions of fluid analyte flow may be stored in a database or look-up table for the purposes of conducting a pattern recognition analysis. It will be appreciated that there are many ways of configuring a particular sensor to respond to a particular fluid analyte flow differently than another sensor. In some examples different sensors may be coated in different materials or may comprise materials such as functionalized graphene that have been functionalized with different functional groups or with different densities of the same functional groups. Differences in the morphology of the sensor may also give rise to differences in the rising and decay sensor signalling in response to a fluid analyte flow with a particular composition.

It will be appreciated that the rising and decay sensor signalling provided by two separate sensors in response to the same modulated fluid analyte flow may be synchronised where the modulated flow arrives at both sensors at the same time. However, in some examples the flow modulator and sensing element may be configured such that the modulated flow arrives at different sensors at different times. In some examples the sensors may be contained within a tube and spaced apart longitudinally along the axis of the tube with a flow modulator at one end of the tube. In such examples some calibration may be required to account for the different arrival times at each sensor of the modulated fluid analyte flow.

In some examples the chemical composition of the fluid analyte flow may vary as it arrives at particular different sensors. This may arise if certain chemical species are selectively adsorbed by the apparatus as the modulated flow passes over the sensing element. Such examples may provide different rising and decay sensor signalling from essentially identical sensors that are spaced apart within the sensing element. Such differences may be exploited to provide different information representative of the composition of the fluid analyte particularly where the characteristics of the selective adsorption are known based on the design or calibration of the apparatus.

Each sensor may be configured to provide the particular detectable rising and decay sensor signalling based on temporally modulated interaction of a particular different chemical species within the fluid analyte flow with the particular sensor. In this way it may be possible to determine the presence of the particular different chemical species within the fluid analyte. In some examples, analysis of the dynamic response of the sensors may also provide information about the concentration of the particular chemical species concerned.

In some examples each sensor may be configured to provide the particular detectable rising and decay sensor signalling with a particular different response time based on temporally modulated interaction of the one or more chemical species within the fluid analyte flow with the particular sensor. Figure 5 illustrates an example of the rising and decay sensor signalling provided by two different sensors with different response times to a fluid analyte with a particular composition. Features of figure 5 that are similar to features of figures 4b and 4c have been given corresponding reference numerals and with not necessarily be discuss in order to improve the clarity of the disclosure.

In response to a particular fluid analyte flow pulse, a first sensor provides a rising sensor signal 540 and a decay sensor signal 542. A portion of the rising sensor signal 540 comprises a saturated sensor signal 546. The same pulse causes the second sensor to provide a different rising sensor signal 550, with a different saturation sensor signal 556 and a different decay sensor signal 552. The example illustrated by figure 5 shows linear responses of the sensor to the fluid analyte flow. However, it will be appreciated that in other examples (not shown) the response of sensor may be highly non-linear. Analysis of the shape of the rising and decay sensor signalling, include the rate of change of the rising signal, the rate of change of the decay signal or the duration of a saturation signal may provide information about the composition of the fluid analyte (looking at the profile response of the sensor signalling). The duration of pulses may vary from milliseconds to tens of seconds depending on the response time a particular sensor.

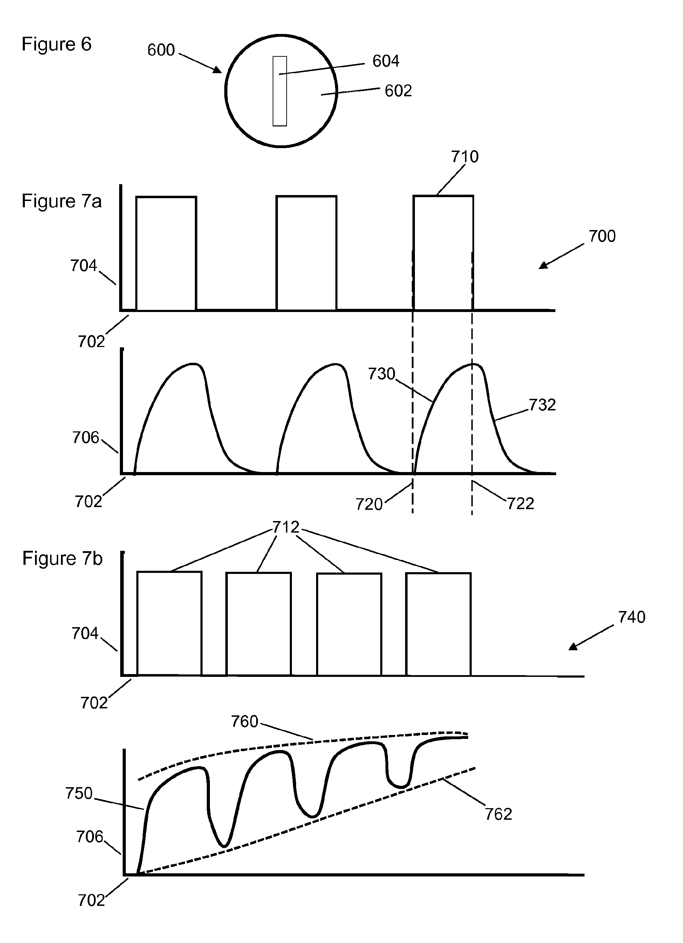

Figure 6a illustrates an example of a fluid analyte flow modulator 600 comprising a micro-electro-mechanical valve. The modulator 600 has an impermeable face 602 through which a fluid analyte may not pass. The modulator 600 has a door 604. When the door 604 is open fluid analyte may flow through the door 604 towards a sensing element (not shown). When the door 604 is shut no fluid analyte may flow through the modulator 600. It will be appreciated that in some examples a modulator may have a plurality of such doors (not shown). In some examples, the mechanism that operates the door 604 may be a microelectro-mechanical system (MEMS). In other examples other suitable types of valve may be used, such as flap valves or ball valves which may be actuated by a suitable control system.

It will be appreciated that a flow of fluid analyte may be provided to a flow modulator in any convenient way, for example by pumping the fluid analyte with a pump or by blowing the fluid analyte with a fan. It will also be appreciated that the sensing element may also be provided with a flow of neutral fluid. Such a flow of neutral fluid may also be modulated and in some examples may be modulated in a manner complementary to the modulated fluid analyte flow. Examples with a neutral fluid flow may advantageously accelerate the removal of the fluid analyte in order to enhance the dynamic response of the sensor to the modulation of the fluid analyte flow. In other examples the fluid analyte flow may be allowed to dissipate without the need for any such neutral fluid flow.

In some examples the fluid analyte flow modulator may be configured to provide for one or more discrete pulses of fluid analyte flow to the sensing element to provide for the temporally modulated interaction of the one or more chemical species of the fluid analyte flow with the sensor element. A discrete pulse may follow a period of time in which there has been no fluid flow. The discrete pulse may then provide a finite amount of fluid flow for a discrete period of time before the flow is cut off and a period of time during which no flow occurs elapses. A period of no flow followed by a discrete pulse may advantageously provide the greatest contrast between a null sensor signal and a rise and decay senor signalling arising from the interaction of the senor and the fluid analyte flow.

In some examples the fluid analyte flow modulator may be configured to provide for a plurality of discrete pulses of fluid analyte flow to the sensing element to provide for a plurality of discrete temporally modulated interactions of the one or more chemical species of the fluid analyte flow with the sensor element, the discrete pulses such that rising and decay sensor signalling from the plurality of the temporally modulated interactions is representative of the composition of the fluid flow. Figure 7a illustrates a series of discrete pulses of fluid analyte flow and the resulting rising and decay sensor signalling 700 provided by a single sensor that interacts with the modulated fluid analyte flow. Time is shown on horizontal axes 702. A first vertical axis 704 shows fluid analyte flow rate and a second vertical axis 706 shows an amplitude of a sensor response to the modulated fluid analyte flow. Discrete pulses of fluid analyte flow are shown as an idealised square wave of fluid analyte flow rate. It will be appreciated that examples of discrete pulses will involve a smoother change of flow rate, but that the idealised square wave provides for a clearer disclosure.

A particular discrete pulse of fluid analyte flow involves a particular fluid analyte flow rate 710. The pulse begins at a first point in time 720 and ends at a second point in time 722. After the first point in time 720, the sensor provides a rising sensor signal 730. After the second point in time 722, the sensor provides a decay sensor signal 732. The particular shape of the rising 730 and decay 732 sensor signal may be representative of the composition of the fluid analyte flow, as discussed above.

In some examples, the fluid analyte flow modulator may be configured to provide for periodic pulses of fluid analyte flow to the sensing element to provide for a plurality of discrete temporally modulated interactions of the one or more chemical species of the fluid analyte flow with the sensor element, the periodic pulses such that a cumulative rising and decay sensor signalling from the plurality of discrete temporally modulated interactions is representative of the composition of the fluid flow. Figure 7b illustrates a series of pulses of fluid analyte flow and the resulting cumulative rising and decay sensor signalling 740. Features similar to features of figure 7a have been given similar reference numerals and will not necessarily be described in order to improve the clarity of the disclosure. It will be appreciated that suitable frequencies for periodic fluid analyte flow modulation may range from 0.01 Hz to 10Hz. In some examples frequencies a high as 100Hz may be advantageous.

In some examples the frequency of periodic pulses, in particular the time between the end of a pulse and the beginning of the next pulse, may be such that a sensor does not have enough time to return to its null output signal before the sensor signalling begins to rise in response to the next pulse. In such examples the sensor signalling will not comprise a periodic series of similar rising and decay sensor signals but will comprise a cumulative rising and decay sensor signal. Figure 7b shows a series of four pulses in fluid analyte flow rate 712 and below them the associated cumulative rising and decay sensor signal 750. It can be seen that the cumulative signal 750 oscillates between an upper envelope 760 and a lower envelope 762. The shape of the cumulative signal 750 and of the upper envelope 760 and of the lower envelope 762 may depend on the composition of the fluid analyte flow. Therefore, analysis of the signalling may provide information about the composition of the fluid analyte flow.

It will be appreciated that different frequencies of pulse may provide different cumulative rising and decay sensor signalling. Therefore, to provide more information about the composition of the fluid analyte some examples apparatus embodiments of the present disclosure may provide a series of periodic pulses of a first frequency followed by a series of periodic pulses of a second different frequency. Other examples may provide a plurality of different series of periodic pulses, each series with a particular different frequency. Particular frequencies may be particularly advantageous for particular sensors that have an appropriate response time. Thus, provision of the plurality of different series of periodic pulses may be especially advantageous where a sensing element comprises a plurality of different sensors.

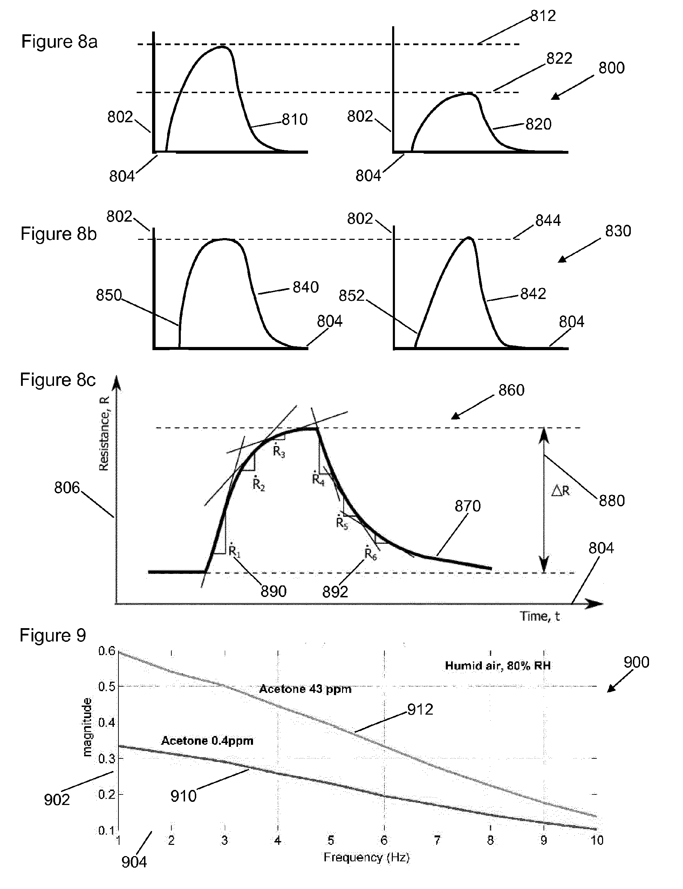

In some examples the rising and decay sensor signalling representative of the composition of the fluid analyte flow may be representative of one or more of presence and concentration of the one or more chemical species. Figure 8a shows the response 800 of a sensor to a pulse of fluid analyte flow of two different fluid analytes with differing compositions. The vertical axes 802 relate to the amplitude of the sensor response and the horizontal axes 804 relate to time. Different characteristic features can be seen.

A first rising and decay sensor signal 810, in response to a pulse of the fluid analyte with a first composition, has a particular shape (characteristic) and reaches a particular maximum amplitude 812 (characteristic maximum amplitude). A second rising and decay sensor signal 820, in response to a pulse of the fluid analyte with a second composition, has a similar shape but reaches a substantially smaller maximum amplitude 822. Depending on the characteristics of the sensor, this difference in amplitude may indicate the presence of a particular chemical species in the second fluid analyte flow that was absent from the first fluid analyte flow. If the relationship between the amplitude of the sensor signal and the concentration of the particular chemical species is known (e.g. based on control experiments) and if the measurement of amplitude can be performed with sufficient accuracy it may be possible to determine the concentration of the particular chemical species in the second fluid analyte. It will be appreciated that the use of a modulated flow of the fluid analyte may be crucial in determining either presence or concentration of a particular chemical species; the presence of a particular chemical species may retard the response of the sensor such that a particular maximum amplitude of sensor signalling is provided. If the same sensor was subject to the fluid analyte for a sufficiently long time then the steady state amplitude may be the same for a broad range of different concentrations, including zero, of the particular chemical species. However, by measuring the dynamic response to a modulated fluid analyte flow this presence or concentration information may be determined.

Figure 8b shows two different rising and decay sensor signals 830 provided by the same sensor in response to two different compositions of a fluid analyte flow. Features of figure 8b that are similar to figure 8a have been given similar reference numerals and will not necessarily be discussed in order to improve the clarity of the disclosure.

The first rising and decay sensor signal 840 has a particular shape, while the second rising and decay sensor signal 842 has a different shape. In this example both signals have approximately the same maximum amplitude 844. However, in this example the gradient of the first signal 840 at the beginning of the sensor response 850 is much larger than the gradient of the second signal 842 at the corresponding part of the sensor response 852. As with the amplitude of the sensor signalling discussed in relation to figure 8a, the gradient of a particular part of the sensor signalling may be used to determine the presence or concentration of a particular chemical species within the fluid analyte flow.

Figure 8c shows the electrical resistance of a sensor as a function of time 860. Resistance is shown on a vertical axis 806 while time is shown on a horizontal axis 804. The rising and decay resistance sensor signal 870 has a particular maximum amplitude response 880. The signal 870 has a particular shape that may be characterised by determining its rate of change over certain parts of the signal. For example the rate of change may be determined over an initial portion 890 of the signal 870 and also over a later part of the signal 892. The rate of change may be characterised by the first time derivative at any particular point. Other information about the shape of the signal may be provided by determining the second or higher time derivatives of the signal. Any such measure of the sensor response may be used to determine information about the presence or concentration of particular chemical species in the fluid analyte flow.

In some examples, extracting information about the presence or concentration of a particular chemical species may require additional information about the fluid analyte flow. For example, it may be necessary to know the temperature or relative humidity of a fluid analyte flow if the sensor response is dependent on such factors. Therefore, in some examples, an apparatus embodiment of the present disclosure may further comprise a temperature sensor and/or a humidity senor or any other sensor.

In some examples the one or more chemical species may comprise one or more of water vapour and one or more of a Volatile Organic Compound (VOC). One example of a VOC is acetone, or propanone. Figure 9 shows a chart 900 with a FFT magnitude of a sensor response shown on a vertical axis 902 and a flow modulation frequency shown on a horizontal axis 904. Measurements relate to a sensor exposed to a modulated fluid analyte flow in which the fluid analyte comprises air with a relative humidity of 80%. A first set of measurements 910 is plotted on the chart 900 and relates to a fluid analyte flow that also comprises 0.4 parts per million (ppm) of acetone. A second set of measurements 912 is plotted on the chart 900 and relates to a fluid analyte flow that also comprises 43 ppm of acetone. The difference between the first set of measurements 910 and the second set of measurements 912 is very pronounced. Such a clear dependence on acetone concentration may be used by example apparatus embodiments to determine the presence or concentration of acetone in the fluid analyte flow.

One possible application of an apparatus embodiment of the present disclosure is a breath sensor for analysing human or animal breath. In humans, the presence of acetone in the breath is a symptom of untreated type 1 diabetes mellitus. Many other diseases or conditions may cause the presence of certain VOC's in breath which may advantageously be identified by using embodiments of the present disclosure.

It will be appreciated that a fluid analyte may be either a liquid analyte or a gaseous analyte, as in the case of breath. The present disclosure may also be relevant for analysing liquid analytes; for example the presence or concentration of certain pollutant chemical species within liquid water may be determined by using embodiments of the present disclosure.

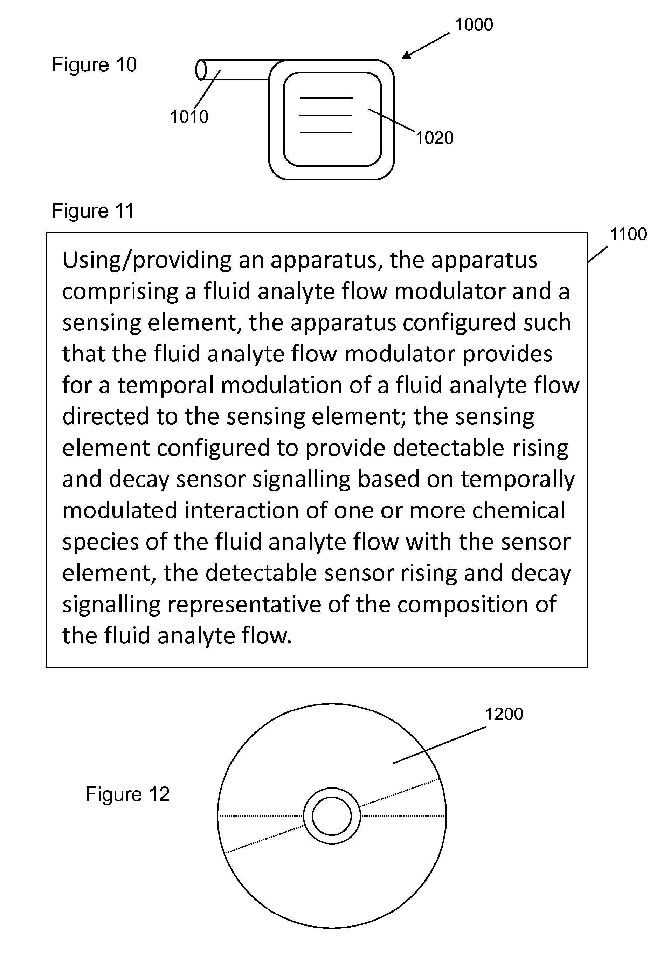

Figure 10 shows an electronic device 1000 comprising an embodiment of the present disclosure. The device 1000 comprises a flow modulator 1010 for providing a modulated flow of fluid analyte to a sensing element (not shown). The device 1000 further comprises a user interface 1020 for providing an output relating to the composition of the fluid analyte to a user. It will be appreciated that some examples may not have a user interface. In some examples, a device may provide information representative of the modulated fluid analyte flow to a server or other remote computer for analysis. In other examples the device may perform the analysis locally.

Many different devices may comprise embodiments of the present disclosure in addition to other functionality. For example a smartphone or Satellite Navigation System may advantageously comprise an apparatus configured to measure the alcohol content of a user's breath such that the device may inform the user as to whether they may legally drive a motor vehicle on a public highway. In some examples a device, such as a smartwatch, may be configured to monitor levels of pollutant chemicals in the atmosphere around a user. If air pollution levels exceed a predetermined threshold the device may be configured to provide an alert to a user. Other examples of electronic devices may include specialist medical devices configured to test a person's breath to identify certain medical conditions. Some examples may be configured to identify any one of a plurality of different medical conditions.

Figure 11 shows a flow diagram illustrating the method 1100 comprising, using/providing an apparatus, the apparatus comprising a fluid analyte flow modulator and a sensing element, the apparatus configured such that the fluid analyte flow modulator provides for a temporal modulation of a fluid analyte flow directed to the sensing element; the sensing element configured to provide detectable rising and decay sensor signalling based on temporally modulated interaction of one or more chemical species of the fluid analyte flow with the sensor element, the detectable sensor rising and decay signalling representative of the composition of the fluid analyte flow.

Figure 12 illustrates schematically a computer/processor readable medium 1200 providing a program according to an example. In this example, the computer/processor readable medium is a disc such as a digital versatile disc (DVD) or a compact disc (CD). In other examples, the computer readable medium may be any medium that has been programmed in such a way as to carry out an inventive function. The computer program code may be distributed between the multiple memories of the same type, or multiple memories of a different type, such as ROM, RAM, flash, hard disk, solid state, etc. The medium may store the computer program which not only detects the signalling, but also interprets the signalling (for example, with reference to control/reference signalling generated from control experiments) in respect of the composition of the fluid analyte flow.

The apparatus shown in the above examples may be a portable electronic device, a laptop computer, a mobile phone, a smartphone, a tablet computer, a personal digital assistant, a navigation device, a watch, a digital camera, a medical device, a non-portable electronic device, a server, a monitor/display, or a module/circuitry for one or more of the same.

Any mentioned apparatus/device/server and/or other features of particular mentioned apparatus/device/server may be provided by apparatus arranged such that they become configured to carry out the desired operations only when enabled, e.g. switched on, or the like. In such cases, they may not necessarily have the appropriate software loaded into the active memory in the non-enabled (e.g. switched off state) and only load the appropriate software in the enabled (e.g. on state). The apparatus may comprise hardware circuitry and/or firmware. The apparatus may comprise software loaded onto memory. Such software/computer programs may be recorded on the same memory/processor/functional units and/or on one or more memories/processors/ functional units.

In some examples, a particular mentioned apparatus/device/server may be pre-programmed with the appropriate software to carry out desired operations, and wherein the appropriate software can be enabled for use by a user downloading a "key", for example, to unlock/enable the software and its associated functionality. Advantages associated with such examples can include a reduced requirement to download data when further functionality is required for a device, and this can be useful in examples where a device is perceived to have sufficient capacity to store such pre-programmed software for functionality that may not be enabled by a user.

Any mentioned apparatus/circuitry/elements/processor may have other functions in addition to the mentioned functions, and that these functions may be performed by the same apparatus/circuitry/elements/processor. One or more disclosed aspects may encompass the electronic distribution of associated computer programs and computer programs (which may be source/transport encoded) recorded on an appropriate carrier (e.g. memory, signal).

Any "computer" described herein can comprise a collection of one or more individual processors/processing elements that may or may not be located on the same circuit board, or the same region/position of a circuit board or even the same device. In some examples one or more of any mentioned processors may be distributed over a plurality of devices. The same or different processor/processing elements may perform one or more functions described herein.

The term "signalling" may refer to one or more signals transmitted as a series of transmitted and/or received electrical/optical signals. The series of signals may comprise one, two, three, four or even more individual signal components or distinct signals to make up said signalling. Some or all of these individual signals may be transmitted/received by wireless or wired communication simultaneously, in sequence, and/or such that they temporally overlap one another.

With reference to any discussion of any mentioned computer and/or processor and memory (e.g. including ROM, CD-ROM etc.), these may comprise a computer processor, Application Specific Integrated Circuit (ASIC), field-programmable gate array (FPGA), and/or other hardware components that have been programmed in such a way to carry out the inventive function.

The applicant hereby discloses in isolation each individual feature described herein and any combination of two or more such features, to the extent that such features or combinations are capable of being carried out based on the present specification as a whole, in the light of the common general knowledge of a person skilled in the art, irrespective of whether such features or combinations of features solve any problems disclosed herein, and without limitation to the scope of the claims. The applicant indicates that the disclosed aspects/examples may consist of any such individual feature or combination of features. In view of the foregoing description it will be evident to a person skilled in the art that various modifications may be made within the scope of the disclosure.

While there have been shown and described and pointed out fundamental novel features as applied to examples thereof, it will be understood that various omissions and substitutions and changes in the form and details of the devices and methods described may be made by those skilled in the art without departing from the scope of the disclosure. For example, it is expressly intended that all combinations of those elements and/or method steps which perform substantially the same function in substantially the same way to achieve the same results are within the scope of the disclosure. Moreover, it should be recognized that structures and/or elements and/or method steps shown and/or described in connection with any disclosed form or examples may be incorporated in any other disclosed or described or suggested form or example as a general matter of design choice. Furthermore, in the claims means-plus-function clauses are intended to cover the structures described herein as performing the recited function and not only structural equivalents, but also equivalent structures. Thus although a nail and a screw may not be structural equivalents in that a nail employs a cylindrical surface to secure wooden parts together, whereas a screw employs a helical surface, in the environment of fastening wooden parts, a nail and a screw may be equivalent structures.

## Claims

1. An apparatus comprising a fluid analyte flow modulator and a sensing element,
the apparatus configured such that the fluid analyte flow modulator provides for a temporal modulation of a fluid analyte flow directed to the sensing element;
the sensing element configured to provide detectable rising and decay sensor signalling based on temporally modulated interaction of one or more chemical species of the fluid analyte flow with the sensor element, the detectable rising and decay sensor signalling representative of the composition of the fluid analyte flow.

2. The apparatus of claim 1, wherein the sensing element is configured to provide the detectable rising and decay sensor signalling based on adsorption of one or more chemical species of the fluid analyte flow by the sensing element.

3. The apparatus of claim 1 or claim 2, wherein the sensing element is configured to provide the detectable rising and decay sensor signalling based on van der Waals' interactions between the sensing element and one or more chemical species of the fluid analyte flow.

4. The apparatus of any preceding claim, wherein the sensing element comprises a plurality of sensors configured to provide a plurality of respective detectable rising and decay sensor signals, each sensor configured to provide a particular detectable rising and decay sensor signalling based on temporally modulated interaction of one or more chemical species of the fluid analyte flow with the particular sensor, the plurality of detectable rising and decay sensor signals representative of the composition of the fluid analyte flow.

5. The apparatus of claim 4, wherein each sensor is configured to provide the particular detectable rising and decay sensor signalling based on temporally modulated interaction of a particular different chemical species within the fluid analyte flow with the particular sensor.

6. The apparatus of claim 4, wherein each sensor is configured to provide the particular detectable rising and decay sensor signalling with a particular different response time based on temporally modulated interaction of the one or more chemical species within the fluid analyte flow with the particular sensor.

7. The apparatus of any preceding claim, wherein the fluid analyte flow modulator is configured to provide for one or more discrete pulses of fluid analyte flow to the sensing element to provide for the temporally modulated interaction of the one or more chemical species of the fluid analyte flow with the sensor element.

8. The apparatus of any preceding claim, wherein the fluid analyte flow modulator is configured to provide for a plurality of discrete pulses of fluid analyte flow to the sensing element to provide for a plurality of discrete temporally modulated interactions of the one or more chemical species of the fluid analyte flow with the sensor element, the plurality of discrete pulses such that rising and decay sensor signalling from the plurality of the temporally modulated interactions is representative of the composition of the fluid flow.

9. The apparatus of any preceding claim, wherein the fluid analyte flow modulator is configured to provide for a plurality of periodic pulses of fluid analyte flow to the sensing element to provide for a plurality of discrete temporally modulated interactions of the one or more chemical species of the fluid analyte flow with the sensor element, the plurality of periodic pulses such that a cumulative rising and decay sensor signalling from the plurality of discrete temporally modulated interactions is representative of the composition of the fluid flow.

10. The apparatus of any preceding claim, wherein the rising and decay sensor signalling representative of the composition of the fluid analyte flow is representative of one or more of presence and concentration of the one or more chemical species.

11. The apparatus of claim 10, wherein the one or more chemical species comprises one or more of water vapour and one or more of a Volatile Organic Compound.

12. The apparatus of any preceding claim, wherein the sensing element comprises:
a thin film of 2D material;
a Field Effect Transistor;
a biosensor; or
one or more of: graphene, graphene oxide, functionalized graphene, and a metal dichalcogenide.

13. The apparatus of any preceding claim, wherein the fluid analyte flow modulator comprises a micro-electro-mechanical valve.

14. A method, the method comprising:
using/providing an apparatus, the apparatus comprising a fluid analyte flow modulator and a sensing element,
the apparatus configured such that the fluid analyte flow modulator provides for a temporal modulation of a fluid analyte flow directed to the sensing element;
the sensing element configured to provide detectable rising and decay sensor signalling based on temporally modulated interaction of one or more chemical species of the fluid analyte flow with the sensor element, the detectable sensor rising and decay signalling representative of the composition of the fluid analyte flow.

15. A computer program, the computer program comprising computer code to detect a signalling produced from an apparatus, the apparatus comprising a fluid analyte flow modulator and a sensing element,
the apparatus configured such that the fluid analyte flow modulator provides for a temporal modulation of a fluid analyte flow directed to the sensing element;
the sensing element configured to provide detectable rising and decay sensor signalling based on temporally modulated interaction of one or more chemical species of the fluid analyte flow with the sensor element, the detectable sensor rising and decay signalling representative of the composition of the fluid analyte flow.
